# EUROPEAN PATENT APPLICATION

(11) **EP 2 311 400 A2**
(43) Date of publication of application: **20.04.2011**
(21) Application number: 10187808.0
(22) Date of filing: 15.10.2010
(51) Int. Cl.: A61B 19/00

(54) **Surgical head clamp**

(30) Priority: 15.10.2009 US 252003 P
(71) Applicant: Hybex Holdings, Inc., Montreal QC H1E 6P1 (CA)
(72) Inventor: Hynes, Brian, Montreal Québec H1E 6P1 (CA)
(74) Representative: Dempster, Benjamin John Naftel

(57) **Abstract**

The invention relates generally to apparatus and devices for stereotactic surgery, computer aided surgery and other similar medical procedures. More particularly, a clamping device (20) for firm attachment to a body part during surgery and for firmly and precisely positioning and orienting medical instruments attached to the clamping device is disclosed.

## Description

### Field of Invention

The invention relates generally to apparatus and devices for stereotactic surgery, computer aided surgery and other similar medical procedures. More particularly, a clamping device for firm attachment to a head and for firmly and precisely positioning and orienting medical instruments attached to the clamping device is disclosed.

### Background of Invention

During stereotactic surgery, intracranial operations, computer aided tomography imaging, or similar medical procedures, it is generally desirable to precisely position and orient various instruments relative to a patient's skull or other anatomy and to hold the patient's head or other anatomy immobilized relative to a support surface, such as an operating table, an operating chair or such other patient support structures.

For example, U.S. Pat. No. 6,117,143 describes an apparatus comprising a head clamp that includes three fixation pins to firmly secure the clamp to a patient's skull, a connector to firmly secure the clamp to a surgical table or like structures, and an articulated arm, including clampable joints, secured to the clamp for attachment of medical or imaging devices.

It is desirable that various parts of a head clamp, once a registration to a neuronavigation system has been completed, do not permit any movement relative to each other, so that registration may be maintained during the medical procedure, such as stereotactic surgery. It is also desirable that the head clamp permits its positioning relative to a patient's head differently to accommodate different procedures to be performed on the patient and that any interference with the performed procedure caused by any attached accessories is minimized.

The forgoing creates challenges and constraints for a clamping device for firm attachment to a head or other body parts and for firmly and precisely positioning and orienting medical instruments attached to the clamping device. There is therefore a need for a clamping device as compared to the existing art. It is an object of the present invention to mitigate or obviate at least one of the above mentioned disadvantages.

### Summary of Invention

The present invention relates to a clamping device for firm attachment to a patient's body during medical procedures and for firmly and precisely positioning and orienting instruments attached to the clamping device. One aspect of the present invention involves a clamping device for firm attachment to a patient's head or other location of the patient's body during surgery or other medical procedures and for firmly and precisely positioning and orienting medical instruments attached to the clamping device. In one embodiment, a clamping device such as a head clamp may generally have a support member such as a clamp arc, body anchoring members such as fixation pins carried by the clamp arc, one or more mounting blocks for attachment of instruments, and securing arrangement to releasably and repositionably secure the one or more mounting blocks to the clamp arc.

In a first aspect of the invention, a clamping device is provided for firm attachment to a body part of a person. The clamping device includes a support member, body anchoring members carried by the support member, and a mounting block releasably secured to the support member. The support member is shaped to accommodate said body part and includes a plurality of connection sites, each connection site having opposed mounting surfaces that are converging toward a narrower side. The body anchoring members engage the body part to secure the support member on the body part such as head in outwardly spaced position therefrom. The mounting block has one or more connection surfaces for attaching medical instruments to the clamping device or for releasably securing said clamping device to a support surface. The mounting block has converging mounting surfaces complementary with the mounting surfaces of the support member for mating therewith, and securing arrangement releasably and repositionably securing the one or more mounting blocks to the support member.

According to one feature of this aspect of the invention, the securing arrangement of the clamping device includes a connector, the connector securing the mounting block to the support member at least one of the plurality of connection sites by applying a force urging relative movement of the mounting block and the support member in a direction for tighter mating between the mounting surfaces of the mounting block and the mounting surfaces of the support member.

According to another feature of this aspect of the invention, at least one of the body anchoring members is a skull fixation pin, the skull fixation pin having a threaded external cylindrical body, and the support member has one or more threaded throughbores to receive the threaded external cylindrical body.

According to a further feature of this aspect of the invention, the clamping device further includes an accessory mounting block and an elevation block releasably secured to the accessory mounting block, the accessory mounting block having converging mounting surfaces to be mated with the outer converging surfaces of the support member.

In another aspect of the invention, a clamping device is provided for firm attachment to a head. The clamping device includes an arc-like support member, a plurality of skull anchoring members carried by said support member for engaging said head to secure said support member on said head in outwardly spaced position therefrom, and a mounting block releasably secured to the support member. The support member has a plurality of threaded throughbores and opposed outer surfaces that are converging toward a narrower side formed at each of the plurality of threaded throughbores. Each of the skull anchoring members has an external threaded cylindrical body sized to be threaded into said threaded throughbores. The mounting block has one or more mounting surfaces for attaching medical instruments to the clamping device or for releasably securing said clamping device to a support surface. The mounting block has inner surfaces to be mated with the outer surfaces of the support member and a thread bore. A shoulder bolt received in one of the threaded througbores of the support member and the threaded bore of the mounting block tightly secures the mounting block to the support member.

According to one feature of this aspect of the invention, the clamping device also has a removal tool, the removal tool having a handle portion, a front portion sized to pass through the threaded throughbores and a threaded body between the handle portion and the front portion, sized to be threaded into the threaded througbores of the support member.

According to another feature of this aspect of the invention, the clamping device has a connection block releasably secured to the support member for securing said clamping device to a support surface, the connection block having converging mounting surfaces to be mated with the converging surfaces of the support member.

In other aspects the invention provides various combinations and subsets of the aspects described above.

### Brief Description of Drawings

For the purposes of description, but not of limitation, the foregoing and other aspects of the invention are explained in greater detail with reference to the accompanying drawings, in which:

Figure **1** shows in a perspective view a clamping device, such as a head clamp, secured to a patient's head; also shown mounted to the head clamp is an articulated, surgical arm for carrying a medical device;

Figure **2** shows in another perspective view a head clamp with the articulated arm removed for better illustration;

Figure **3** shows in a cross-sectional view the connection between a mounting block and the head clamp arc;

Figure **4** shows a mounting block in a perspective view;

Figure **5** shows in a perspective view an accessory mounting block and an elevation block secured to the accessory mounting block;

Figure **6** shows in a perspective view an accessory mounting block;

Figure **7** shows in a perspective view an installation/removal tool for installing and removing a mounting block;

Figures **8A, 8B** and **8C** show in cross-sectional views alternative configurations for attaching a mounting block to a head clamp arc; and

Figure **9** shows in a perspective view a head clamp with a differently constructed accessory mounting block and an elevation post.

### Detailed Description of Embodiments

The description which follows and the embodiments described therein are provided by way of illustration of an example, or examples, of particular embodiments of the principles of the present invention. These examples are provided for the purposes of explanation, and not limitation, of those principles and of the invention. In the description which follows, like parts are marked throughout the specification and the drawings with the same respective reference numerals.

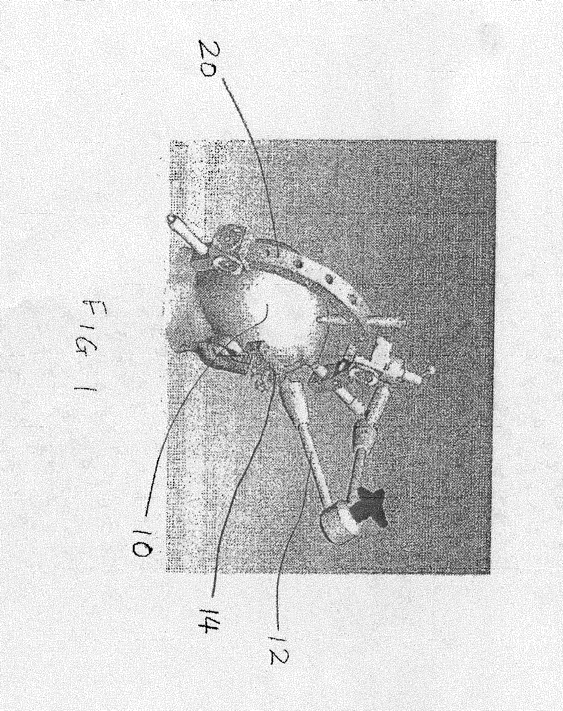

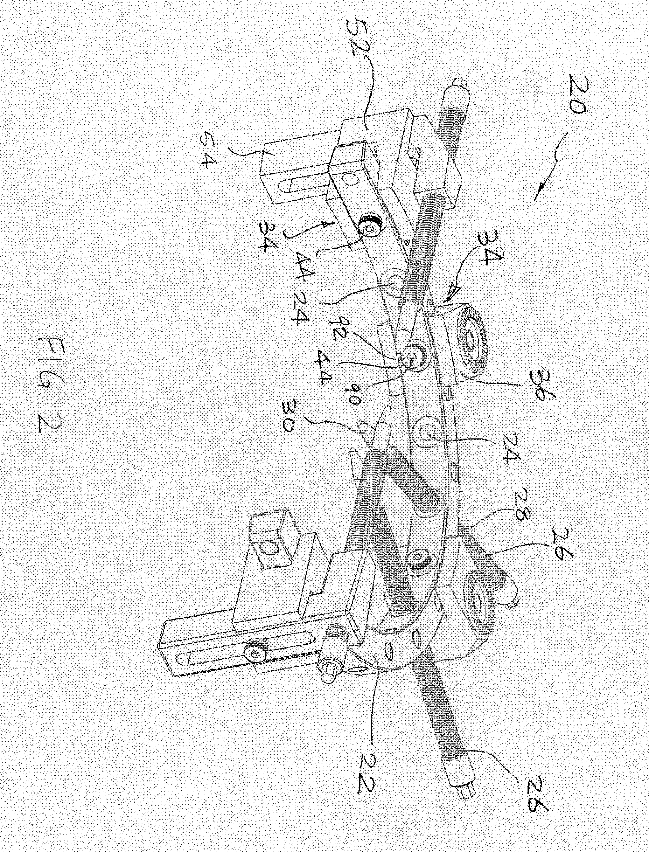

In the description reference may be made to the general environment of a clamping device. By way of a general overview, Figure **1** shows in a perspective view a clamping device such as head clamp **20** firmly attached to a patient's head **10.** Mounted to the head clamp is an articulated, surgical arm **12** for carrying a medical device, such as probe **14.** Figure **2** shows in another perspective view a head clamp **20** like that shown in Figure **1****,** with the articulated arm **12** removed for better illustration.

Head clamp **20** has a support member for carrying body anchoring members and mounting blocks. The support member is shaped to accommodate a patient's head (or an animal's head) or other parts of a patient's body (e.g., leg, arm or torso), and may be arc-like. Body anchoring members, such as fixation pins, fix the support member relative the patient's head, for example, and places the support member in spaced position outwardly from the head. Figures **1** and **2** illustrate an arc-like support member, namely a head clamp arc **22.** Head clamp arc **22** can have a generally circular shape. The circular shape allows the arc to be oriented in any suitable position to suit the needs of performed procedure. Head clamp arc is suitably sized to fit human and/or animal skull sizes. It will be appreciated, however, that the support member may also take other shapes, such as a ring, a plate having an inner curved edge etc., to accommodate a head, a leg, an arm, among others.

Head clamp arc **22** has a plurality of threaded throughbores, namely, threaded throughholes **24,** spaced from each other along the arc and formed generally along a radial direction, to accept skull fixation pins **26.** Each skull fixation pin **26** has an externally threaded cylindrical body **28** that can be threaded into one of the threaded throughholes **24** and a sharp pin tip **30** mounted to the cylindrical body for engaging skull **10.** Of course, for engaging other body parts, such as leg or torso, fixation pins may be replaced by fixation pads as body anchoring members, to reduce discomfort that might be caused by a pin but still effectively maintain the base support member in a fixed relationship to the engaged body part.

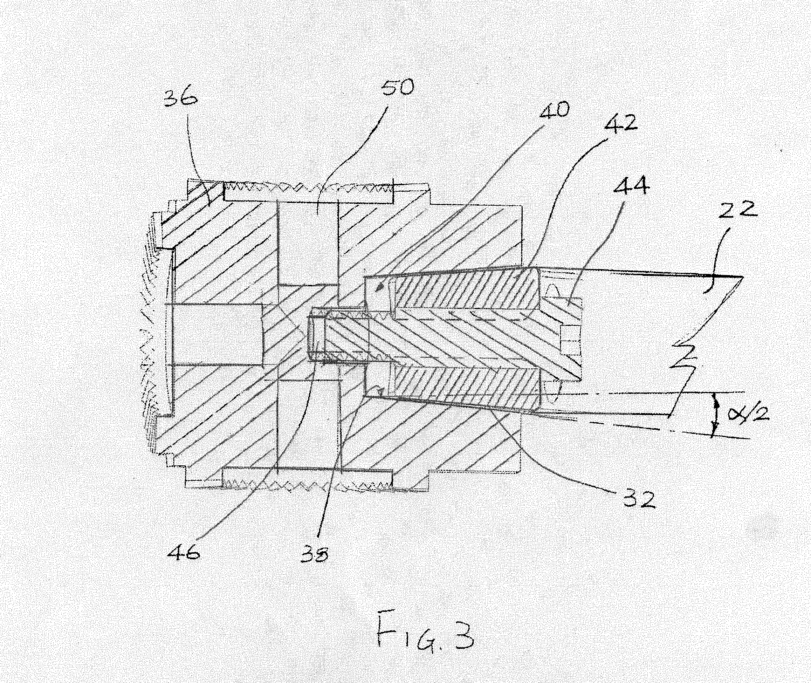

Head clamp arc **22** has opposed outer surfaces **32,** formed at connection sites **34** surrounding each threaded throughholes (more clearly shown in Figure **3****).** As can be seen more clearly in Figure **3****,** a radial outward portion of head clamp arc **22** has a cross-sectional shape that is essentially trapezoidal in a radial plane, with outer surfaces of the trapezoidal arc converging radially outwardly. In other words, a portion of the outer surfaces **32,** namely the portion forming the trapezoidal shape, converge radially outwardly. It will be understood, however, that the cross section of the head clamp arc may not always be trapezoidal. It may take a different shape. In general, the cross section includes a portion of converging outer surfaces. While the trapezoidal shape shown in Figure **3** has outer surfaces converging radially outwardly, the converging surfaces may also converge inwardly. Further, conveniently, the outer surfaces may extend over the entire length of the arc, such as shown in Figure **2****.**

Referring to Figures **2****,** **3** and **4****,** head clamp **20** includes one or more mounting blocks **36** for mounting to the head clamp arc **22** at connection sites **34.** Mounting block **36** has opposing inner surfaces **38** to be mated with the outer surfaces of the head clamp arc **22** at a connection site. Inner mounting surfaces **38** have at least a converging portion matching the converging portion of the outer mounting surfaces **32.** Figure **3** shows a mounting block **36** that has a trapezoidal slot **40** matching the trapezoidal cross-sectional shape **42** of the head arc clamp, with matching and complementary mounting surfaces. These matching mounting surfaces, i.e., the complementary, converging outer surfaces **32** of head clamp arc **22** and inner surfaces **38** of the trapezoidal slot **40** of mounting block **36** allow the mounting block **36** to be firmly attached to the head clamp arc **22,** as will be further described now. As can be seen in Figure **3****,** both trapezoidal cross sections have narrower end located further outwardly of the center. This allows the mounting block to be located on the outside of the head clamp arc **22.** A securing arrangement is provided to force the mounting block toward the head clamp arc, or more generally, to force the mounting block and the head clamp arc toward each other, so that the mounting block is releasably secured to the head clamp arc when needed. A shoulder bolt **44,** for example, may be used for pulling the mounting block toward the head clamp arc. A threaded hole **46** is formed in the mounting block **36.** The shoulder bolt **44** is sized smaller than the threaded throughhole **24** of the head clamp arc so it can pass through the threaded throughhole **24** unhindered and threaded into the threaded hole **46** of the mounting block to pull the mounting block toward the head clamp arc. The shoulder bolt **44** pulls the mounting block toward the head clamp arc **22** until the mounting block **36** cannot be pulled any closer to the head clamp arc **22** and will lock the mounting block onto the head clamp arc. Any translational movement of the mounting block **36** is restricted by the shoulder bolt **44** and the matching converging surfaces. Any rotational movement of the mounting block rotate relative to the head clamp arc is restricted by tight engagement of matching converging surfaces of the mounting block with those of head clamp arc. As will be understood and as will be illustrated in some examples to be provided later, a variety of methods may be employed to force the mounting block and the head clamp arc toward each other. Such securing arrangement also is not limited to mechanical configurations. For example, the mounting block or head clamp arc **22** may be magnetized (or selectively magnetized by electricity) for pulling the mounting block toward the head clamp arc.

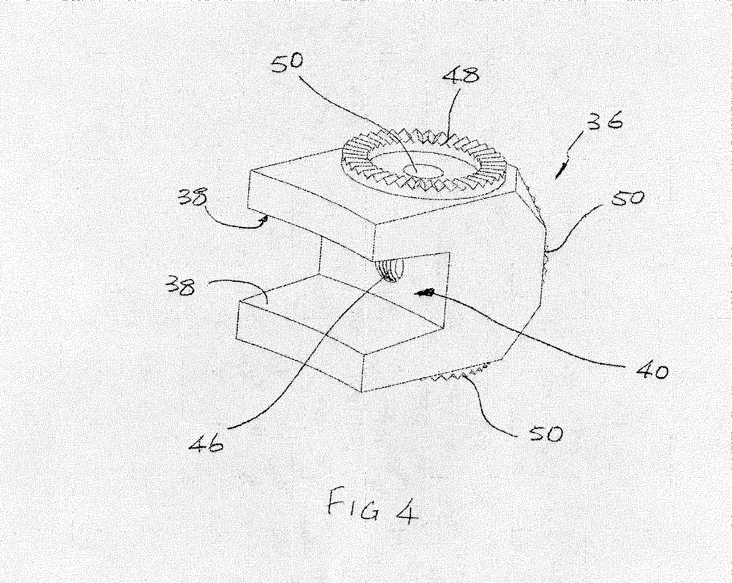

Referring to Figures **2** and **4****,** mounting block **36** has one or more outer connection surfaces formed thereon for connection of medical instrument or accessories to the head clamp. Mounting block **36** may be a starburst connection block with toothed connection surfaces **48** and a central hole as shown in Figure **4****.** The starburst connection block may be used to mount a variety of devices with mating toothed connection surfaces. A central hole **50** is formed near the center of the toothed connection surfaces **48.** The central hole **50** may be threaded, in which may be threaded a bolt or screw, or a throughhole for a bolt to pass therethrough, to securely attach a device to the starburst connection block. The cooperating toothed connection surfaces of the mounting block and the mounted devices also encourage precisely locating the connected device at one of the angular positions defined by the toothed connection surfaces. One such mating device or attachment may be an articulated surgical arm **12** to be attached to support instruments used in surgical procedures. Another such attachment may be a marker for providing a reference point during neurovavigation. The starburst connection block also allow mounting of other attachments, such as an attachment to hold the mounting block in a fixed position relative to an operating table, operating chair, desk or other mounting support structure or surface. This would hold a patient's head immobilized for surgery or other procedures.

As noted, a surgeon may need to locate the head clamp **20** to different positions relative to the head or orient differently for different procedures while minimizing interference of the head clamp with the procedure performed. The medical instrument or devices attached to the head clamp may also need to be located differently relative to the head clamp arc **22** as required by different procedures. This can be accomplished by threading skull fixation pins **26** into different threaded throughholes **24,** or repositioning the mounting block or blocks to different connection sites, or connecting the instruments or devices to different toothed connection surfaces of the mounting block or blocks. To reposition a mounting block **36,** the shoulder bolt **44** is removed to allow separation of the mounting block from the header clamp arc. A special tool, as will be fully described below, may be provided for facilitating the separation of the mounting block form the header clamp arc.

There may also be situations where a skull fixation pin **26** may still interfere with the procedure performed even when it is moved to a different threaded throughhole **24.** It is therefore desirable that skull fixation pins may be secured to head clamp arc **22** at a location generally spaced from the plan defined by head clamp arc. Similarly, attachments secured to a mounting block may interfere with performed procedures and moving attachments to a location spaced from the plan defined by head clamp arc may minimize the interference.

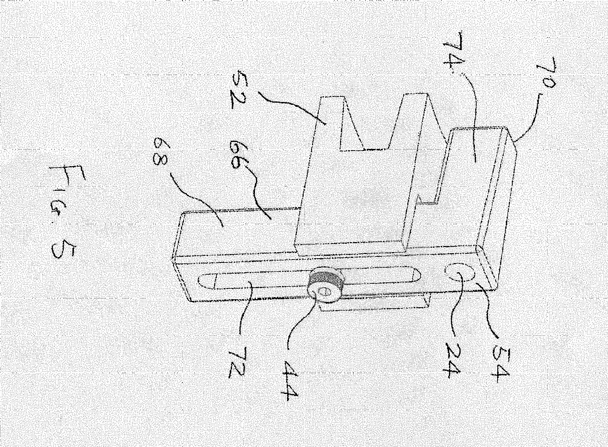

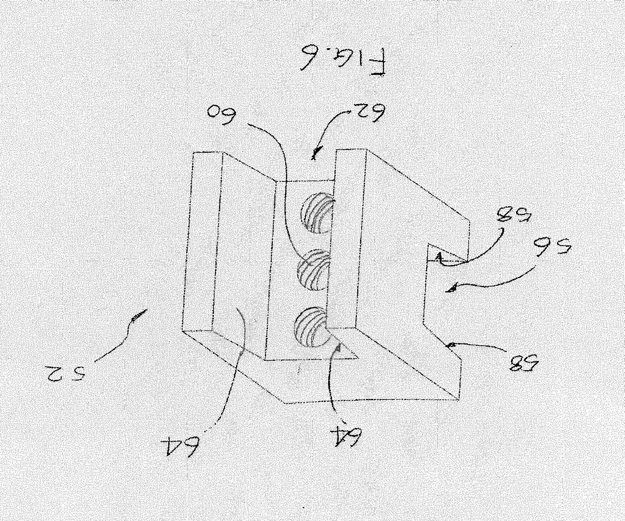

Figure **5** shows an accessory mounting block **52** and an elevation block **54** secured to the accessory mounting block. As can be more clearly seen in Figure **6****,** accessory mounting block **52** has a front slot **56** that has front converging surfaces **58** for mating with the outer surfaces of head clamp arc **22.** Several threaded throughbores **60** are provided so that a shoulder bolt **44** inserted from the inside radius of the head clamp arc **22** can pass through a throughhole 24 and thread into one of the threaded throughbores **60** to secure the accessory mounting block to head clamp arc. Accessory mounting block also has a rear slot **62** that has opposing, rear converging surfaces **64** for accepting elevation block 54.

Referring to Figure **5****,** elevation block **54** has an elongated body **66** sized to be partially received in the rear slot and slidable along the rear slot **62.** The elongated body has opposed outer converging surfaces **68** that converge toward its front side **70.** The outer converging surfaces of the elevation block are to be mated with the rear converging surfaces **64** of the accessory mounting block **52** when a section of the elongated body is received in the rear slot. After the elevation block is suitably positioned relative to the accessory mounting block, a shoulder bolt **44** is passed through a slot **72** formed in the elongated body **66,** threaded into one of the threaded throughbores **60,** and tightened to pull the elevation block toward the accessory mounting block until they cannot be moved closer toward each other. The elevation block **54** is thus locked in place and immovably secured to the accessory mounting block **52.** Moving the elevation block to another location relative to the accessory mounting block is done by unscrewing the shoulder bolt securing the elevation block to the accessory mounting block and thereby allowing their separation and tightening the shoulder bolt again after the elevation block is appropriately repositioned.

Elevation block has a mounting site **74** formed integral with the elongated body **66.** Mounting site **74** may be configured to accept a skull fixation pin, or for connection of attachment thereto. For example, a threaded throughhole **24** may be formed at the mounting site **74** for accepting a skull fixation pin. The threaded throughhole **24** may be oriented generally parallel to the plane defined by the head clamp arc **22** or angled with respect to the plane. The mounting site may also have one or more toothed connection surfaces 48 like that of mounting block 36, formed on side surfaces at mounting site 74 or front side 70, for attaching medical instrument or connection of attachment.

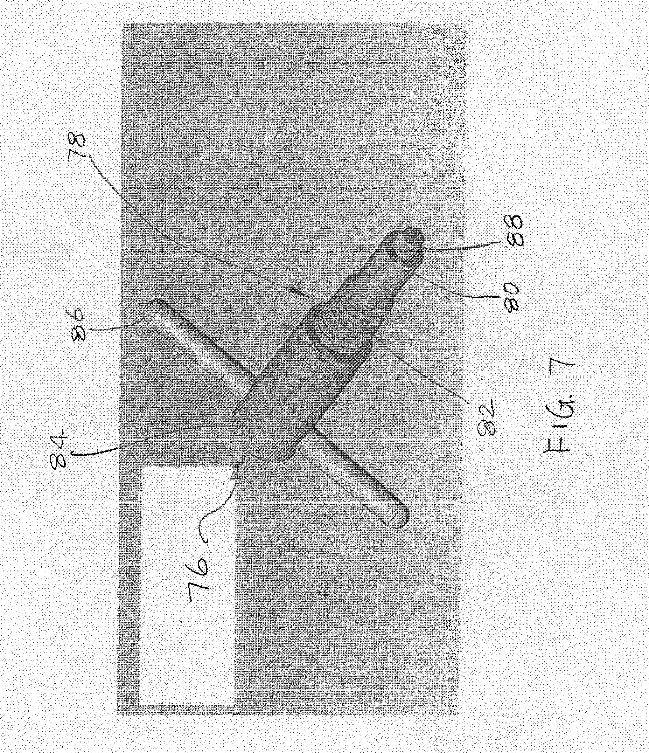

Conveniently, a combined installation and removal tool **76** is provided for easy installation and removal of skull fixation pins. Figure **7** illustrates such a combined installation and removal tool **76,** which has an elongated cylindrical body **78,** which includes a front portion **80** and a threaded body portion **82,** and a handle portion **84** for fixing a handle **86** thereto. The threaded body portion **82** is sized to be threaded into a threaded throughhole **24** of the head clamp arc **22.** The front portion **80** is sized to pass through the threaded throughholes **24** unhindered. The front portion **80** does not need to be threaded and is sized for pushing starburst mounting block **36** away from the head clamp arc **22.** For example, the front portion may be sized larger than the threaded hole **46** of the mounting block. Alternatively, the front portion may be sized smaller than the threaded hole **46** but sufficiently long so that the front portion (or its tip **88)** can reach the bottom of the threaded hole **46.** The tip of the front portion may have a non-cylindrical shape (such as a hex tip **88** or a tip of any other suitable shape) that fits into a complementarily shaped hole (such as a hex hole **90)** formed on the bolt head 92 of the shoulder bolt **44,** so that the installation and removal tool can also be used for tightening or loosening the shoulder bolt.

To removal a starburst mounting block or accessory mounting block, the shoulder bolt is first loosened and removed, using the combined installation and removal tool **76,** for example. It will be appreciated that when a mounting block is tightened onto the head clamp arc, the converging surfaces tend to lock the mounting block onto the arc and it may be difficult to separate the starburst mounting block from the head clamp arc. Conveniently, the installation and removal tool can be threaded into the threaded throughhole until the front portion **80** is in contact with the bottom of the threaded hole **46** of the starburst mounting block **36** and pushes the starburst mounting block **36** off of the head clamp arc.

As will be appreciated, the converging nature of the converging surfaces of the mounting block and the head clamp arc assists the tight engagement of the mounting block with the head clamp arc and prevents their relative movement once the mounting block is tightened and locked in place. The converging angle, namely the angle **α** between the converging surfaces, is generally in the range of **5** to **40** degrees. Depending or materials used and whether the contacting surfaces of the mounting block and the clamp arc are formed using the same material, the converging angle may be outside this range. However, to obtain satisfactory results, the converging angle should not be significantly larger than **60** degrees or much smaller than **2** degrees. As will also be appreciated, the converging angle may be evenly or unevenly divided between the pair of converging surfaces of the slot or arc. Figure **3** shows the converging angle to be evenly divided, but that is not necessary. It is found that a converging angle of about **10** degrees, divided evenly between the pair of converging surfaces, tends to provide satisfactory results with mounting block and head clamp both made of steel.

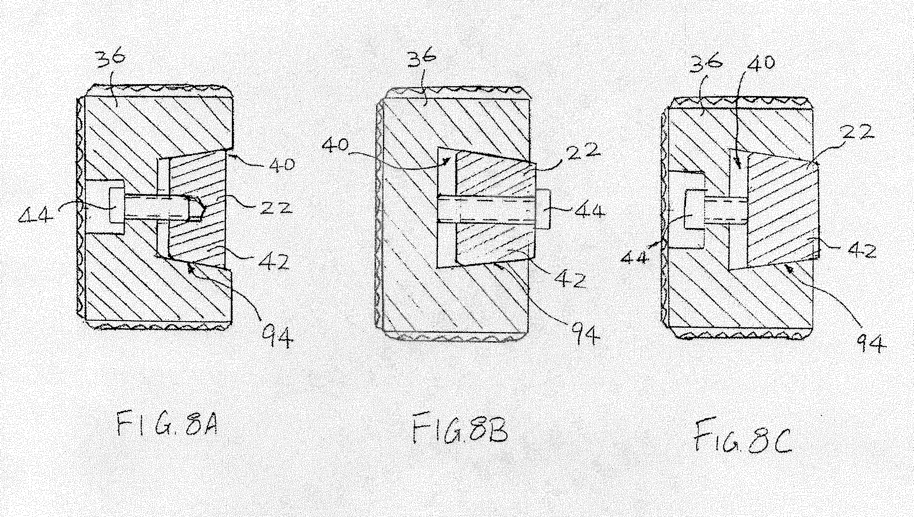

As noted earlier, there are different ways of configuring the converging surfaces and the engagement arrangement to tighten the mounting block, other than that is shown in Figure **3****.** For example, Figure **8A** is a cross-sectional view of a mounting block **36** and a head clamp arc **22** at a connection site **34** (see Figure **2**) illustrating one such alternative configuration. The mounting block has a trapezoidal slot **40** that matches the trapezoidal cross-sectional shape **42** of the head clamp arc. The converging surfaces **94** converge outwardly along a radial direction. Unlike that shown in Figure **3****,** the shoulder bolt passes through a throughhole of the mounting block and is threaded into the threaded hole of the head clamp arc. Tightening the shoulder bolt **44** pulls the head clamp arc **22** towards the mounting block **36** to lock the mounting block onto the head clamp arc. Figure **8B** shows in a cross-sectional view another alternative configuration. The converging surfaces **94** converge inwardly along a radial direction. A shoulder bolt **44** is threaded into a threaded throughhole of the head clamp arc **22** and pushes the mounting block **36** away from the head clamp arc to tightly lock the mounting block on the head clamp arc. Similarly, Figure **8C** shows in a cross-sectional view yet another alternative configuration. The converging surfaces **94** converge inwardly along a radial direction, as in Figure **8****B.** The shoulder bolt **44** is threaded through a threaded throughhole of the mounting block **36** and pushes the head clamp arc **22** away from the mounting block, in order to lock the mounting block tightly onto the head clamp arc. These are but a few examples to illustrate different configurations of engagement arrangement, for tightly locking the mounting block onto the head clamp arc. The same configurations may be applied to accessory mounting blocks, too. Other configurations are also possible. For example, it is not necessary to use a threaded shoulder bolt. Magnetic force can be utilized to force a mounting block towards or away from a head clamp arc. Such magnetic force also can be selectively applied, for example, by utilizing electromagnet.

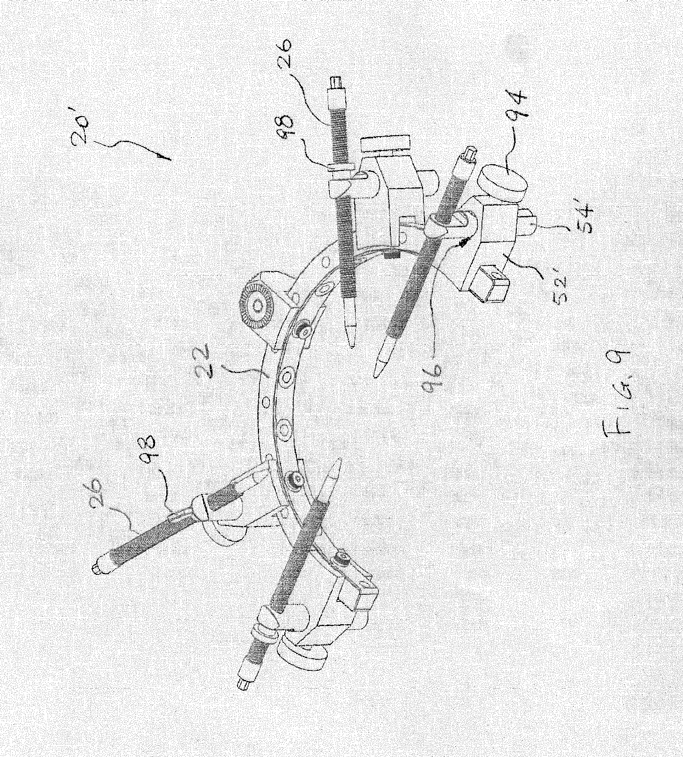

Figure **9** shows in a perspective view a head clamp **20'** with a differently constructed accessory mounting block **52'** and elevation block **54'.** Unlike accessory mounting block **52** shown in Figure **6****,** which provides an open rear slot **62** for receiving an elongated body **66** of elevation block **54,** the differently constructed accessory mounting block has a throughhole **96** defined therein. Elevation block **54** has the form of a post. Elevation post **54'** is slidably received in throughhole **96.** Elevation post **54'** and its matching throughhole may have a cylindrical shape as shown in Figure **9** or any other suitable shape. A skull fixation pin **26** is threaded in a threaded throughhole **24** provided on elevation post **54',** to carry the pin **26** with the elevation post **54'** as the elevation post slides along the throughhole **96.** A locking nut **98,** such as a wing nut or a thumbnut, may be provided to releasably lock pin **26** relative to the elevation post **54'.** A locking thumbscrew **94,** or any other suitable locking screw, is provided to releasably secure the elevation post relative to the accessory mounting block **52'.** To minimize damages to the elevation block **52',** the tip of the locking thumbscrew may be made from a plastic material.

Various embodiments of the invention have now been described in detail. Those skilled in the art will appreciate that numerous modifications, adaptations and variations may be made to the embodiments without departing from the scope of the invention. Since changes in and or additions to the above-described best mode may be made without departing from the scope of the invention, the invention is not to be limited to those details but only by the appended claims.

## Claims

1. A clamping device for firm attachment to a body part of a person, the clamping device comprising:
a support member, the support member being shaped to accommodate said body part and including a plurality of connection sites, each connection site having opposed mounting surfaces that are converging toward a narrower side,
body anchoring members carried by said support member for engaging said body part to secure said support member on said body part in outwardly spaced position therefrom,
a mounting block releasably secured to the support member at one of said plurality of connection sites, the mounting block having one or more connection surfaces for attaching one or more medical instruments to the clamping device or for releasably securing said clamping device to a support surface, the mounting block having converging mounting surfaces complementary with the mounting surfaces of the support member for mating therewith, and
securing arrangement releasably and repositionably securing the one or more mounting blocks to the support member.

2. The clamping device of claim 1, further comprising a connection block releasably secured to the support member for securing said clamping device to the support surface, the connection block having converging mounting surfaces to be mated with the mounting surfaces of at least one of the plurality of connection sites.

3. The clamping device of claim 1, wherein the securing arrangement includes a connector, the connector securing the mounting block to the support member at least one of the plurality of connection sites by applying a force urging relative movement of the mounting block and the support member in a direction for tighter mating between the mounting surfaces of the mounting block and the mounting surfaces of the support member.

4. The clamping device of claim 3, wherein the securing arrangement includes a throughbore formed at one of the at least one connection site and the mounting block and a threaded bore formed at the other of the at least one connection site and the mounting block, and the connector is a shoulder bolt sized to pass through the throughbore and threaded in the threaded bore.

5. The clamping device of claim 3, wherein the securing arrangement includes a threaded throughbore formed at one of the at least one connection site and the mounting block and the connector is a shoulder bolt sized to be threaded in the threaded thoughbore and rested against the other of the at least one connection site and the mounting block.

6. The clamping device of claim 1, wherein the mounting block is releasably secured to the support member by magnetic force.

7. The clamping device of claim 1, wherein at least one of the body anchoring members is a skull fixation pin, the skull fixation pin having a threaded external cylindrical body, and wherein the support member has one or more threaded throughbores to receive the threaded external cylindrical body.

8. The clamping device of claim 7, further comprising an accessory mounting block and an elevation block releasably secured to the accessory mounting block, the accessory mounting block having converging mounting surfaces to be mated with the outer converging surfaces of the support member.

9. The clamping device of claim 8, wherein the elevation block has an elevated threaded throughbore to receive the threaded external cylindrical body.

10. The clamping device of claim 8, wherein the elevation block has one or more accessory connection surfaces for attaching one or more medical instrument.

11. A clamping device for firm attachment to a head, the clamping device comprising:
an arc-like support member, the support member having a plurality of threaded throughbores, the support member having opposed outer surfaces that are converging toward a narrower side formed at each of the plurality of threaded throughbores,
a plurality of skull anchoring members carried by said support member for engaging said head to secure said support member on said head in outwardly spaced position therefrom, each of said skull anchoring members having an external threaded cylindrical body sized to be threaded into said threaded throughbores,
a mounting block releasably secured to the support member, the mounting block having one or more mounting surfaces for attaching one or more medical instruments to the clamping device or for releasably securing said clamping device to a support surface, the mounting block having inner surfaces to be mated with the outer surfaces of the support member and a thread bore; and
a shoulder bolt to be received in one of the threaded througbores of the support member and the threaded bore of the mounting block for tightly securing the mounting block to the support member.

12. The clamping device of claim 11, further comprising a removal tool, the removal tool having a handle portion, a front portion sized to pass through the threaded throughbores and a threaded body between the handle portion and the front portion, sized to be threaded into the threaded througbores of the support member.

13. The clamping device of claim 11, further comprising a connection block releasably secured to the support member for securing said clamping device to the support surface, the connection block having converging mounting surfaces to be mated with the converging surfaces of the support member.

14. The clamping device of claim 11, further comprising an accessory mounting block releasably secured to the support member, the accessory mounting block having converging mounting surfaces to be mated with the converging mounting surfaces of the support member, and an elevation block releasably secured to the accessory mounting block.

15. The clamping device of claim 14, wherein the elevation block has an elevated threaded throughbore to receive the threaded external cylindrical body.

16. The clamping device of claim 14, wherein the elevation block has one or more elevated connection surfaces for attaching one or more medical instrument.

17. The clamping device of claim 14, wherein the accessory mounting block and the elevation block have complementary converging connection surfaces for securing the elevation block to the accessory mounting block.

18. The clamping device of claim 14, wherein the accessory mounting block has a throughhole defined therein and the elevation block has an elongated body slidably received in said throughhole, said claiming device further comprising a locking screw for releasably securing said elevation block to said accessory mounting block.

19. The clamping device of claim 11, further comprising one or more locking nuts for releasably locking respective one or more external threaded cylindrical bodies to the support member.
